(19) Europäisches Patentamt
European Patent Office
Office européen des brevets

(11) Publication number: 0 095 562
B1

(12) **EUROPEAN PATENT SPECIFICATION**

(45) Date of publication of patent specification: **01.06.88**

(51) Int. Cl.[4]: **B 01 J 31/12,** C 07 C 41/02, C 07 B 41/04

(21) Application number: **83102321.3**

(22) Date of filing: **09.03.83**

(54) **Catalysts for alkoxylation reactions.**

(30) Priority: **01.06.82 US 383387**

(43) Date of publication of application:
**07.12.83 Bulletin 83/49**

(45) Publication of the grant of the patent:
**01.06.88 Bulletin 88/22**

(84) Designated Contracting States:
**BE DE FR GB SE**

(56) References cited:
**GB-A-2 021 606**
**US-A-3 029 216**
**US-A-3 321 533**
**US-A-3 395 185**
**US-A-3 719 636**

(73) Proprietor: **Vista Chemical Company**
**15990 North Barkers Landing Road**
**Houston Texas 77224 (US)**

(72) Inventor: **Yang, Kang**
**2501 Robin Road**
**Ponca City, OK 74601 (US)**

(74) Representative: **Patentanwälte Grünecker, Kinkeldey, Stockmair & Partner**
**Maximilianstrasse 58**
**D-8000 München 22 (DE)**

**The file contains technical information submitted after the application was filed and not included in this specification**

EP 0 095 562 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the production of alkoxylated organic compounds by reacting said compounds with an alkoxylating agent in the presence of a Lewis type acid catalyst to yield a very sharply peaked alkoxylate distribution.

In general, the reaction of a variety of organic materials together with an adducting material such as ethylene oxide or propylene oxide to form alkoxylated materials is well known in the art. US—A—2,683,087 discloses that water adsorption by paper articles is improved by the use of amine adducts of ethylene oxide. GB—A—847,714 teaches the processing of prehydrolyzed sulfate wood pulp into viscose by incorporating a propylene oxide/ethylene oxide adduct of ethylene diamine. FR—A— 1,122,729 discloses the use of an acylarylpolyglycol adduct to the viscose pulp or slurry. BE—A—555,529 discloses an anti-static agent for synthetic fibers produced by esterifying one mole of lauric acid with one mole of an ethoxylated glycerol. GB—A—763,215 suggests an ethoxylated organic sulfamide as an anti-static agent for textiles.

GB—A—705,117 discloses an emulsifier combination for pesticides comprising a mixture including a tall oil or dodecyl mercaptan adduct. Polyhydric alconol ethoxylates find uses in foods and feeds as shown by US—A—2,674, 534 which discloses the use of sorbitol laurate and sorbitol oleate adducts in the coating of ice cream bars. Alkylene oxide adducts are also used in the leather industry in formulations for tanning, dyeing, and lubricating leather. Adducts of organic materials also have a variety of uses and metal working industries where ester, ether and amine adducts are the products used most frequently. Ethylene oxide adducts such as sorbitan monostearate adducts have been found useful in pharmaceutical and cosmetic preparations and are used to provide activities such as drug carriers, emulsifiers and solubilizers. Ethylene oxide adducts of nonyl phenols have been used to produce detergents and cleaning agents, domestic and industrial laundry detergents, detergent builders, polishers, sanitizers, and dry cleaning materials. Alkyl phenol adducts are especially good soil suspending materials when used in detergent formulations since they possess excellent detergency, fat emulsifying power, concentration effect, chemical resistance, hard water stability and pH properties.

Much literature is available in the general area of alcohol alkoxylation. These references relate to the catalytic ability of various materials in the mechanism of kinetics of these reactions. For example, FR—A—1,365,945 teaches the use of compounds containing an active hydrogen atom reacted with ethylene oxide in the presence of an alkali metal base.

Both basic and acidic catalysts in general are known to produce alkoxylation of organic materials. However, alkoxylation of these materials invariably produces a distribution of various adducts. For example, in surfactant applications, an adduct of too few ethylene oxide molecules is not effective because of poor solubility. In contrast, an adduct with too many ethylene oxide molecules is likewise undesirable because surface tension reduction per unit mass decreases drastically as the molecular weight increases. Thus it has long been essential to produce and use alkoxylates with as sharp a distribution in the desired mole adduct range for the particular use of the material as can possibly be realized.

Normally, acid catalyzed reactions produce such alkoxylates, but these catalysts produce harmful side products and must be separated and removed prior to use. Base catalysts normally do not produce by-products of acidic catalysts but provide a much broader distribution of alkoxylation adducts, thus making them economically unsuitable. Thus both methods have disadvantages.

Therefore, it would be desirable to provide a catalyst system for the alkoxylation of organic materials which provides low by-product levels typical of base catalysts, yet provides a narrow distribution of the preferred mole adducts normally obtained from acid catalysts. Such a catalyst would promote the narrowing of product distribution curve and would contribute significantly to the intrinsic value of the alkoxylate produced.

Such a catalyst is described in US—A—4,239,917 and 4,306,093. However, these catalysts, while effective in producing a very sharply peaked distribution product, do not produce as much peaking as the catalysts to be described herein.

The use of dialkyl aluminium fluoride or alkyl aluminium difluoride is known as a catalyst for the polymerization of epoxides to produce polyalkoxy alcohols as described in US—A—3,029,216 and 3,313,743. However, these catalysts were not used in the alkoxylation of alcohols and require water, none of which is necessary in the present invention. In addition, dialkylaluminum halides or alkyl aluminum dihalides can be used to produce ethoxylated alcohols using different methods such as the polymerization of ethylene oxide as described in US—A—3,321,533. However in this process the materials are not used as catalysts, but rather as reactants since sodium hydroxide acts as the ethoxylation catalyst.

US—A—3,395,185 utilizes organoaluminum zinc compounds as catalysts in the preparation of low molecular weight polyoxymethylene glycols. Zinc, however, was not an effective catalyst in the present invention. US—A—2,716,137 uses nitrogen-containing catalysts. These materials are characterized by low reaction rates and objectionable odors. US—A—4,282,387 uses catalysts such as calcium, strontium and barium acetates and naphthenates. These materials produce alkoxylate products more sharply peaked than commercially used basic catalysts such as sodium and potassium hydroxide, but do not provide the extremely high peaking of the present invention.

The ethoxylation of alcohols using aluminum compounds such as aluminum trifluoride or trialkyl aluminum is described in US—A— 2,870,220; 3,350,462; 3,719,636 and 3,969,417.

US—A—3,719,636 discloses a method of preparing biodegradable glycidol surfactants by reacting an aliphatic alcohol with glycidol in the presence of a polar, non-reactive, organic solvent and a basic or acid catalyst. The acid catalyst may be any of the Lewis type acids, with the preferred catalyst being boron fluoride etherate.

EP—A—0043963 discloses a process for the ethoxylation of primary alcohols which comprises in a first stage reacting ethylene oxide with a mixture of primary monohydric alcohols in the presence of an acid catalyst consisting of Friedel-Crafts type catalysts, sulphuric acid, and phosphoric acid to prepare an adduct of said alcohol containing 1 to 6 moles of ethylene oxide and in a second stage reacting ethylene oxide with the neutralised and washed reaction product from the first stage in the presence of an alkaline catalyst to prepare a stable, liquid nonionic surface active agent. The acid catalyst used in the first stage of said process can be one of the known Friedel-Crafts type reaction catalysts, such as the fluorides and chlorides or boron, aluminum, iron, tin and titanium, and complexes of such halides with ethyl ether. However, as is obvious from all the Examples, the preferred catalyst is boron trifluoride monoetherate. The reaction mixture in the first stage of said process is maintained at a temperature within the range from about 20°C to 80°C.

It is therefore the task underlying the present invention to provide an alkoxylation method which will yield a narrow alkylene oxide adduct distribution in the alkoxylation of certain organic materials, namely alcohols, alkyl phenols, aldehydes, ketones, amides, amines, organic acids and mercaptans while providing low levels of undesirable by-products and non-desired alkoxylation adduct.

According to the invention, this task is solved by an alkoxylation method comprising:

contacting under alkoxylating conditions at a temperature between about 20°C and about 260°C for a time sufficient to alkoxylate to the extent desired,

an alkoxylate precursor selected from the group consisting of polyhydric alcohols, unsaturated alcohols, linear and branched saturated alcohols, alkyl phenols, aldehydes, ketones, amides, amines, organic acids and mercaptans; and

an alkoxylating agent selected from the group consisting of alpha and beta alkylene oxides; in the presence of a Lewis type acid catalyst, characterised in that said catalyst comprises at least one catalyst component of the general formula

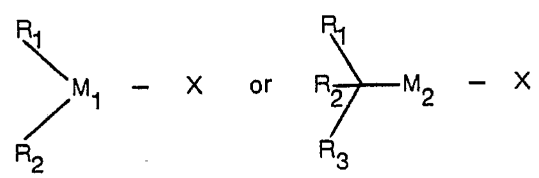

wherein $M_1$ is selected from the group consisting of aluminum, gallium, indium and thallium,

$M_2$ is selected from the group consisting of titanium, zirconium and hafnium,

X is a halogen selected from the group consisting of chlorine and fluorine and

$R_1$, $R_2$ and $R_3$ are, independently, selected from the group consisting of hydrogen, alkyl groups containing from 1 to 20 carbon atoms, alkoxide groups containing from 1 to 20 carbon atoms and halogen, wherein at least one of $R_1$, $R_2$ or $R_3$ must be an alkyl or an alkoxide group.

Representative but non-exhaustive examples of such catalysts are dialkylaluminum fluorides, alkylaluminum difluorides, trialkyl zirconium fluorides, dialkyl zirconium difluorides, alkyl zirconium trifluorides, trialkyl titanium fluorides, dialkyl titanium fluorides, alkyl titanium difluorides, dialkoxy aluminum fluorides, alkoxy aluminum difluorides, trialkoxy zirconium fluorides, dialkoxy zirconium difluorides, alkoxy zirconium trifluorides, trialkoxy titanium fluorides, dialkoxy titanium difluorides, alkoxy titanium trifluorides, dialkoxy gallium fluorides, dialkoxy indium fluorides, dialkoxy thallium fluorides, and trialkoxy hafnium fluorides. These alkyl and alkoxy groups will normally contain from about 1 to about 20 carbon atoms, but the preferred catalysts are those containing from about 1 to about 14 carbon atoms.

The method of the invention is carried out at temperatures of from about 20°C to about 260°C. However, more normal temperatures range from about 90°C to about 200°C. For practical purpoes, most commercial operations will be carried out in the temperature range of from about 100°C to about 200°C.

The method of the present invention can be carried out at ambient pressure. However, pressures above or below ambient can be carried out as desired. Pressure or lack of pressure is not a critical factor in the present invention and pressures may be used as convenient. Normally pressures of up to about 6.89 kPa (100 psig) can be used, but pressures below about 4.14 kPa (60 psig) are preferred. It is simply more convenient to normally carry out the reactions in the pressure range of from about atmospheric to about 6.89 kPa (100 psig).

The alkoxylations of the present invention are carried out with materials or mixtures of materials comprising alpha and beta alkylene oxide. Of these materials, ethylene oxide, propylene oxide or mixtures of these are preferred.

The reaction products can have any desired content of adducting material. For example, an alcohol alkoxylations ethylene oxide will normally comprise from about 30 to about 90% of product content based

on weight. However, for most purposes the content of ethylene oxide will range from about 40% to about 70% by weight. The weight of adducting material present in the reaction is not critical other than the minimum amount necessary to provide sufficient units to reach the mole adduct level desired for the materials to be reacted.

For practical purposes, normally from about 0.05 to about 5.0 weight percent catalyst based upon the weight of the material to be reacted is present in the reaction. These catalysts are effective in the absence of promoters or cocatalysts, although promoters or cocatalysts can be used. Preferred levels of catalysts in the reaction mixture are from about 0.1 to about 1.0% by weight based on the total reaction mixture weight.

The catalysts used in the method of the present invention are normally added to the reaction mixture in a solution form. However, in order to render these catalysts less air sensitive and more stable, catalysts can optionally be supported on materials having active surface hydroxyl groups. Representative but non-exhaustive examples of such supports are alumina, diatomaceous earth, silica, bentonite glass, and various clays.

Examples of organic materials which can be alkoxylated by the method of the invention include

a) alcohols having a boiling point above 100°C and containing a total of 2 to 30 carbon atoms; and having 2 or more hydroxyl containing compounds of the general formula

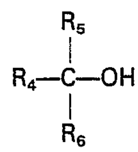

wherein $R_4$, $R_5$, and $R_6$ are, independently, linear or branched acyclic groups, alicyclic groups, aryl groups, cyclic groups, or hydrogen and wherein the R-designated groups can in addition contain one or more functional groups selected from the group consisting of amine, carboxyl, halogen, nitro, carbonyl, and amide;

b) aldehydes and ketones having boiling points above 100°C and containing a total of from 2 to 30 carbon atoms, and having one or more carbonyl containing compounds of the general formula

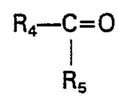

wherin $R_4$ and $R_5$ have the same meanings as stated above and wherein the R-designated groups can in addition contain one or more functionalities selected from the group consisting of carboxyl, hydroxyl, halogen, nitro, amine, or amide;

c) primary, secondary or tertiary amides having a boiling point of above 100°C and containing a total of from 1 to 30 carbon atoms and containing 1 or more amide containing compounds of the general formula

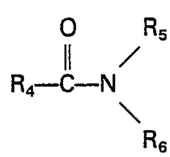

wherein $R_4$, $R_5$, and $R_6$ have the same meanings as stated above and wherein the R-designated groups can in addition contain one or more other functionalities selected from the group consisting of hydroxyl, carboxyl, carbonyl, amine, nitro, or halogen;

d) primary, secondary or tertiary amines having a boiling point above 100°C, containing from a total of 1 to 30 carbon atoms and containing 1 or more amine containing compounds of the general formula

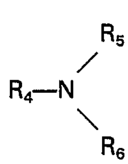

wherein $R_4$, $R_5$ and $R_6$ have the same meanings as above and wherein the R-designated groups can in addition contain one or more functionalities selected from the group consisting of hydroxyl, carbonyl, halogen, carboxyl, nitro or amide;

e) organic acids having a boiling point of above 100°C, containing from a total of 1 to 30 carbon atoms and having 1 or more carboxylic acid containing compounds of the general formula

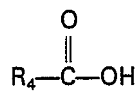

wherein $R_4$ has the same meaning as stated above and wherein the R group can in addition contain one or more functionalities selected from the group consisting of carbonyl, hydroxyl, halogen, nitro, amine, or amide;

f) alkyl phenols having a boiling point of above 100°C, containing a total of from 6 to 30 carbon atoms and having 1 or more compounds of the general formula

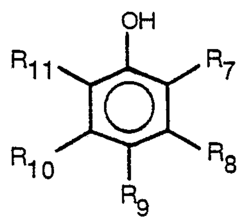

wherein $R_7$, $R_8$, $R_9$ and $R_{10}$ and $R_{11}$ are, independently, hydrogen, halogen, hydroxyl, nitro, or carbonyl, linear or branched acyclic groups, alicyclic groups cyclic groups, aryl groups, or substituted aryl groups and wherein in addition the R-designated groups can contain one or more functionalities selected from the group consisting of halogen, ether, nitro, carboxyl, carbonyl, amine, amide, or hydroxyl;

g) mercaptans of the general formula

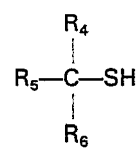

wherein $R_4$, $R_5$ and $R_6$ have the same meanings as stated above, containing from 1 to 30 carbon atoms and wherein the $R_4$, $R_5$ and $R_6$-designated groups can in addition contain one or more functionalities selected from the group consisting of carboxyl, hydroxyl, halogen, nitro amine, or amide, and

h) alcohols of the general formula

$$ROH$$

where R is a linear or branched alkyl group containing from 1 to 30 carbon atoms, an aryl group or a cyclic group containing from 6 to 30 carbon atoms, or an olefinic or acetylenic group containing from 2 to 30 carbon atoms.

While the method of the invention is effective with all classes of alcohols, both saturated and unsaturated, saturated alcohols are preferred. Of these alkanols are most preferred. The alkanols primary, secondary linear and branched, linear and branched primary alkanols are the most commonly used and are the preferred materials for alkoxylation using the method of the present invention.

Representative but non-exhaustive examples of alcohols which can be alkoxylated according to the present invention are 1-dodecanol; 1-tridecanol; 1-tetradecanol; 1-pentadecanol; 1-hexadecanol; 1-heptadecanol; 1-octadecanol; 1-nonadecanol; 1-eicosanol; 1-docosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 1-pentyl-1-tridecanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol; 2-tetradecanol; 3-tetradecanol; 4-tetradecanol; 5-tetradecanol; 6-tetradecanol; 7-tetradecanol; 2-hexadecanol; 3-hexadecanol; 4-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol; 9-octadecanol-1; 2,4,6-trimethyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 2-methyl-2-undecanol; 3-methyl-3-undecanol; 4-methyl-4-undecanol; 2-methyl-2-tridecanol; 3-methyl-3-tridecanol; 4-methyl-3-tridecanol; 4-methyl-4-tridecanol; 3-ethyl-3-decanol; 3-ethyl-3-dodecanol; 2,4,6,8-tetramethyl-2-nonanol; 2-methyl-3-undecanol; 2-methyl-4-undecanol; 4-methyl-2-undecanol; 5-methyl-2-undecanol; 4-ethyl-2-decanol; 4-ethyl-3-decanol; tetracosanol; hexacosanol; octacosanol; triacontanol; dotriacontanol; hexatriacontanol; 2-decyl-tetradecanol; 2-dodecylhexadecanol; 2-tetradecyloctadecanol; 2-hexadecyleicosanol, and unsaturated

alcohols such as 1-hexyn-3-ol; 4-ethyl-1-octyn-3-ol; 2-methyl-3-butyn-2-ol; 3-methyl-1-pentyn-3-ol; oleyl alcohol (technically named cis-9-octadecene-1-ol); 2,5-dimethyl-4-octyne-3,6-diol; 2,4,7,9-tetramethyl-n-decyne-4,7-diol; 3-dodecene-1-ol; and 3,6-dimethyl-8-dodecene-1-ol.

Representative but non-exhaustive examples of various polyols which can be alkoxylated according to the present invention are:

ethylene glycol
1,2-propylene glycol
1,4-butanediol
1,6-hexanediol
1,10-decanediol
1,3-butylene glycol
diethylene glycol
diethylene glycol monobutyl ether
diethylene glycol monomethyl ether
diethyl glycol monoethyl ether
dipropylene glycol
dipropylene glycol monomethyl ether
ethylene glycol monomethyl ether
ethylene glycol monoethyl ether
ethylene glycol monobutyl ether
hexylene glycol
mannitol
sorbitol
pentaerythritol
dipentaerythritol
tripentaerythritol
trimethylolpropane
trimethylolethane
neopentyl glycol
diethaholamine
triethanolamine
diisopropanolamine
triisopropanolamine
1,4-dimethylolcyclohexane
2,2-bis(hydroxymethyl)propionic acid
1,2-bis(hydroxymethyl)benzene
4,5-bis(hydroxymethyl)furfural
4,8-bis(hydroxymethyl)tricyclo[5,2,1,0] decane
tartaric acid
2-ethyl-1,3-hexanediol
2-amino-2-ethyl-1,3-propanediol
triethylene glycol
tetraethylene glycol
glycerol
ascorbic acid

Representative but non-exhaustive examples of various aldehydes and ketones which can be alkoxylated according to the present invention are

lauryl aldehyde
benzaldehyde
2-undecanone
acetophenone
2,4-pentandione
acetylsalicylic acid
ortho-chlorobenzaldehyde
para-chlorobenzaldehyde
cinnamic aldehyde
diisobutyl ketone
ethylacetoacetate
ethyl amyl ketone
camphor
para-hydroxybenzaldehyde
2-carboxybenzaldehyde
4-carboxybenzaldehyde
salicylaldehyde
octyl aldehyde

decyl aldehyde
p-methoxybenzaldehyde
p-aminobenzaldehyde
phenylacetaldehyde
acetoacetic acid
2,5-dimethoxybenzaldehyde
1-naphthyl aldehyde
terephthaldehyde

Representative but non-exhaustive examples of amides which can be alkoxylated according to the instant invention are:

formamide
benzamide
acetanilide
salicylamide
acetoacetanilide
ortho-acetoacetotoluidide
acrylamide
N,N-diethyltoluamide
N,N-dimethylacetamide
N,N-dimethylformamide
phthalimide
octylamide
decylamide
laurylamide
stearylamide
N,N-dimethylollaurylamide
N,N-dimethylacrylamide
para-chlorobenzamide
para-methoxybenzamide
para-aminobenzamide
para-hydroxybenzamide
ortho-nitrobenzamide
N-acetyl-para-aminophenol
2-chloroacetamide
oxamide
N,N-methylene-bis-acrylamide

Representative but non-exhaustive examples of amines which can be alkoxylated according to the present invention are:

aniline
benzylamine
hexadecylamine
triphenylamine
aminoacetic acid
anthranilic acid
cyclohexylamine
tert-octylamine
ortho-phenylenediamine
meta-phenylenediamine
para-phenylenediamine
N-acetyl-para-aminophenol
2-amino-4-chlorophenol
2-amino-2-ethyl-1,3-propanediol
ortho-aminophenol
para-aminophenol
para-aminosalicylic acid
benzyl-N,N-dimethylamine
tert-butylamine
2-chloro-4-aminotoluene
6-chloro-2-aminotoluene
meta-chloroaniline
ortho-chloroaniline
para-chloroaniline
4-chloro-2-nitroaniline
cyclohexylamine
dibutylamine

2,5-dichloroaniline
3,4-dichloroaniline
dicyclohexylamine
diethanolamine
N,N-diethylethanolamine
N,N-diethyl-meta-toluidine
N,N-diethylaniline
diethylenetriamine
diisopropanolamine
N,N-dimethylethanolamine
N,N-dimethylaniline
2,4-dinitroaniline
diphenylamine
ethyl-para-aminobenzoate
N-ethylethanolamine
N-ethyl-1-naphthylamine
N-ethyl-ortho-toluidine
N-ethylaniline
ethylenediamine
hexamethylenetetraamine
2,4-lutidine
N-methylaniline
methyl anthranilate
p,p′-diaminodiphenyl methane
ortho-nitroaniline
para-nitroaniline
tert-octylamine
piperazine
ethanolamine
isopropanolamine
ortho-toluidine
para-toluidine
2,4-tolyenediamine
triethanolamine
tributylamine
triisopropanolamine
2,4-dimethylxylidine
para-methoxyaniline
nitrilotriacetic acid
N-phenyl-1-naphthylamine

Representative but non-exhaustive examples of organic acids which can be alkoxylated according to the present invention are:

formic acid
acetic acid
valeric acid
heptanoic acid
2-ethylhexanoic acid
lauric acid
stearic acid
oleic acid
tall oil acids
hydrogenated tall oil acids
benzoic acid
salicyclic acid
adipic acid
azelaic acid
fumaric acid
citric acid
acrylic acid
aminoacetic acid
para-aminosalicylic acid
anthranilic acid
butyric acid
propionic acid
ricinoleic acid

chloroacetic acid
ortho-chlorobenzoic acid
2,4-dichlorophenoxyacetic acid
tert-decanoic acid
parap-aminobenzoic acid
abietic acid
itaconic acid
lactic acid
glycolic acid
malic acid
maleic acid
cinnamic acid
para-hydroxybenzoic acid
methacrylic acid
oxalic acid
myristic acid
palmitic acid
tert-pentanoic acid
phenylacetic acid
mandelic acid
sebacic acid
tallow fatty acids
hydrogenated tallow fatty acids
tartaric acid
trichloroacetic acid
2,4,5-trichlorophenoxyacetic acid
undecylenic acid
crotonic acid
pelargonic acid
acetoacetic acid
para-nitrobenzoic acid
ascorbic acid
nitrilotriacetic acid
naphthenic acids
1-naphthoic acid
trimellitic acid

Representative but non-exhaustive examples of various phenols which can be alkoxylated according to the present invention are

phenol
ortho-cresol
meta-cresol
para-cresol
2,4-dimethylphenol
2,5-dimethylphenol
2,6-dimethylphenol
ortho-chlorophenol
meta-chlorophenol
para-chlorophenol
para-nitrophenol
para-methoxyphenol
salicylic acid
meta-hydroxyacetophenone
para-aminophenol
ortho-phenylphenol
nonylphenol
octylphenol
t-butyl-para-cresol
hydroquinone
catechol
resorcinol
pyrogallol
1-naphthol
2-naphthol
4,4'-isopropylidenediphenol (bisphenol A)
methyl salicylate

benzyl salicylate
4-chloro-2-nitrophenol
para-t-butylphenol
2,4-di-t-amylphenol
2,4-dinitrophenol
para-hydroxybenzoic acid
8-hydroxyquinoline
methyl para-hydroxybenzoate
2-nitro-para-cresol
ortho-nitrophenol
para-phenylphenol
phenyl salicylate
salicylaldehyde
p-hydroxy benzaldehyde
2-amino-4-chlorophenol
ortho-aminophenol
salicylamine

The invention is more concretely described with reference to the examples below wherein all parts and percentage are by weight unless otherwise specified.

Example 1

Diethylaluminum fluoride (DEAF) was used as an ethoxylation catalyst. ALFOL 1214 alcohol (a 12—14 carbon atom alcohol, trademark of and sold by CONOCO Inc.) in the amount of 300 grams was mixed with 10 cubic centimeters 25% diethylaluminum fluoride in heptane. The mixture was degassed for 30 minutes at 150°C with nitrogen purging at a rate of 500 cubic centimeters per minute. After evacuation, 64 grams of ethylene oxide was introduced at about 2.76 kPa (40 psig) pressure at 150°C. The reaction mixture was cooled to 100°C whereafter 5 grams of calcium hydroxide was added. Fifteen minutes agitation followed at which time the reaction mixture was filtered. Distribution of the product alkoxylated alcohol was determined by using high pressure liquid partition liquid chromatography. The distribution is shown in Figure 1.

Comparative Example 1

As a comparative example a known $BF_3$/etherate catalyst was tested. 300 grams of an ALFOL 1214 alcohol was mixed with 2 cubic centimeters $BF_3$ etherate. The mixture was heated to 50°C and was evacuated briefly at this temperature as an ethoxylating agent 64 grams of ethylene oxide was introduced at about 0.207 kPa (3 psig) at 50—60°C. The distribution was again determined using high pressure liquid partition chromatography and the results are set forth in Figure 1.

Comparative Example 2

A strontium hydroxide nonyl phenol promoted catalyst as described in US—A— 4,223,164 was prepared by mixing 67,857 grams of ALFOL 1214 alcohol, 55,071 grams of nonylphenol and 15,003 grams of strontium hydroxide. 5 grams of resulting catalyst was mixed with 300 grams of ALFOL 1214 alcohol and a reaction was carried out at 170°C and at 2.76 kPa (40 psig). After introducing 64 grams of ethylene oxide the product was cooled to 100°C a neutralized with carbon dioxide. The distribution was determined using high pressure liquid partition chromatography. The results are set forth in Figure 1.

Example 2

A mixture of 50 cubic centimeters of methanol and 10 cubic centimeters of 25% diethylaluminum fluoride in hexane was refluxed 30 minutes, then rotary dried to remove excess methanol. The catalyst together with 300 grams of ALFOL 1214 alcohol was purged with nitrogen at 500 cubic centimeters per minute for 30 minutes at a temperature of 150°C. After a brief evacuation 64 grams of ethylene oxide was introduced at 2.76 kPa (40 psig) and 150°C and allowed to react for 135 minutes. The distribution was obtained using high pressure liquid partition chromatography. Distribution is set forth in Figure 2.

Example 3

The effectiveness of support on the catalyst was determined, by mixing 5 grams of silica gel with 10 cubic centimeters of 25% diethylaluminum fluoride in hexane. The mixture was rotary dried to remove hexane. The resulting catalyst was used with 300 grams of ALFOL 1214 alcohol and the ethoxylation carried out as described in Example 2. As an alkylating agent 118 grams of ethylene oxide was introduced over 280 minutes. The product contained 5.2% unreacted alcohol.

A comparative experiment was carried out using $BF_3$ to produce the product with the same free alcohol level. The results are summarized in Figure 3, clearly showing the higher peaking obtained using the silica supported diethyl aluminum fluoride catalyst.

Comparative Example 3

An experiment as in Example 1 is performed with 2 grams of dibutyl zinc. No measurable ethoxylation occurred.

## Claims

1. An alkoxylation method, comprising:

contacting under alkoxylating conditions at a temperature between about 20°C and about 260°C for a time sufficient to alkoxylate to the extent desired,

an alkoxylate precursor selected from the group consisting of polyhydric alcohols, unsaturated alcohols, linear and branched saturated alcohols, alkyl phenols, aldehydes, ketones, amides, amines, organic acids and mercaptans; and

an alkoxylating agent selected from the group consisting of alpha and beta alkylene oxides; in the presence of a Lewis type acid catalyst, characterised in that said catalyst comprises at least one catalyst component of the general formula

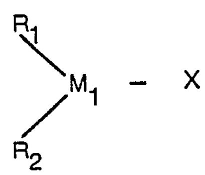

or

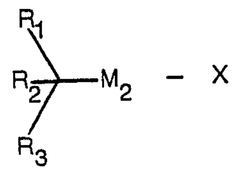

wherein $M_1$ is selected from the group consisting of aluminum, gallium, indium and thallium,

$M_2$ is selected from the group consisting of titanium, zirconium and hafnium,

X is a halogen selected from the group consisting of chlorine and fluorine and

$R_1$, $R_2$ and $R_3$ are, independently, selected from the group consisting of hydrogen, alkyl groups containing from 1 to 20 carbon atoms, alkoxide groups containing from 1 to 20 carbon atoms and halogen, wherein at least one of $R_1$, $R_2$ or $R_3$ must be an alkyl or an alkoxide group.

2. The alkoxylation method of claim 1 wherein X is fluorine.

3. The alkoxylation method of claims 1 or 2 wherein said catalyst is selected from the group of catalyst components consisting of dialkylaluminum fluorides, alkylaluminum difluorides, trialkyl zirconium fluorides, dialkyl zirconium difluorides, alkyl zirconium trifluorides, trialkyl titanium fluorides, dialkyl titanium fluorides, alkyl titanium difluorides, dialkoxyaluminum fluorides, alkoxyaluminum difluorides, trialkoxy zirconium fluorides, dialkoxy zirconium difluorides, alkoxy zirconium trifluorides, trialkyoxy titanium fluorides, dialkoxy titanium difluorides, and alkoxy titanium trifluorides, wherein said alkyl and alkoxy groups contain from 1 to 14 carbon atoms.

4. The alkoxylation method of claims 1 to 3 wherein said catalyst is diethyl aluminum fluoride.

5. The alkoxylation method of claims 1 to 4 wherein said catalyst is a supported catalyst produced by contacting said catalyst component of said general formula with a catalyst support having active surface hydroxyl groups.

6. The alkoxylation method of claim 5 wherein said catalyst support is selected from the group consisting of silica, alumina, diatomaceous earth, bentonite glass and various clays.

7. The alkoxylation method of claim 6 wherein said catalyst support is silica and said catalyst component is diethyl aluminum fluoride.

8. The alkoxylation method of claims 1 to 7 wherein the alkoxylating agent is selected from the group consisting of ethylene oxide, propylene oxide and mixtures thereof.

9. The alkoxylation method of claims 1 to 8 wherein said contacting is conducted at a temperature between about 90°C and about 200°C.

10. The alkoxylation method of claims 1 to 9 wherein said alkoxylate precursor is an alcohol.

## Patentansprüche

1. Alkoxylierungsverfahren, umfasend:

Kontaktieren unter Alkoxylierungsbedingungen bei einer Temperatur zwischen etwa 20°C und etwa 260°C für eine Zeit, die ausreichend ist, um bis zum erwünschten Ausmaß zu alkoxylieren,

eines Alkoxylat-Vorläufers, gewählt aus der Gruppe mehrwertige Alkohole, ungesättigte Alkohole, lineare und verzweigte, gesättigte Alkohole, Alkylphenole, Aldehyde, Ketone, Amide, Amine, organische Säuren und Mercaptane; und

eines Alkoxylierungsmittels, gewählt aus der Gruppe α- und β-Alkylenoxide; in Gegenwart eines Säurekatalysators vom Lewis-Typ, dadurch gekennzeichnet, daß der Katalysator mindestens eine Katalysatorkomponente der allgemeinen formel umfaßt:

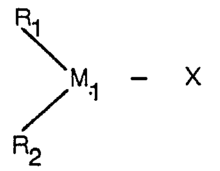 oder 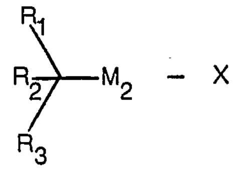

worin bedeuten:

$M_1$ gewählt aus der Gruppe Aluminium, Gallium, Indium und Thallium,

$M_2$ gewählt aus der Gruppe Titan, Zirkon und Hafnium,

X Halogen, gewählt aus der Gruppe Chlor und Fluor und

$R_1$, $R_2$ und $R_3$ unabhängig voneinander gewählt aus der Gruppe Wasserstoff, Alkylgruppen mit 1 bis 20 Kohlenstoffatomen atomen, Alkoxidgruppen mit 1 bis 20 Kohlenstoffatomen und Halogen, wobei mindestens eines von $R_1$, $R_2$ oder $R_3$ eine Alkyl- oder Alkoxidgruppe sein muß.

2. Alkoxylierungsverfahren nach Anspruch 1, wobei X Fluor ist.

3. Alkoxylierungsverfahren nach Anspruch 1 oder 2, wobei der Katalysator gewählt wird aus der Gruppe von Katalysatorkomponenten, bestehend aus Dialkylaluminiumfluoriden, Alkylaluminiumdifluoriden, Trialkylzirkonfluoriden, Dialkylzirkondifluoriden, Alkylzirkontrifluoriden, Trialkyltitanfluoriden, Dialkyltitanfluoriden, Alkyltitandifluoriden, Dialkoxyaluminiumfluoriden, Alkoxyaluminiumdifluoriden, Trialkoxyzirkonfluoriden, Dialkoxyzirkondifluoriden, Alkoxyzirkontrifluoriden, Trialkoxytitanfluoriden, Dialkoxytitandifluoriden und Alkoxytitantrifluoriden, wobei die Alkyl- und Alkoxygruppen 1 bis 14 Kohlenstoffatome enthalten.

4. Alkoxylierungsverfahren nach den Ansprüchen 1 bis 3, wobei der Katalysator Diethylaluminiumfluorid ist.

5. Alkoxylierungsverfahren nach den Ansprüchen 1 bis 4, wobei der Katalysator ein trägerhaltiger Katalysator ist, hergestellt durch Kontaktieren der Katalysatorkomponente der allgemeinen Formel mit einem Katalysatorträger, der aktive Oberflächenhydroxylgruppen besitzt.

6. Alkoxylierungsverfahren nach Anspruch 5, wobei der Katalysatorträger gewählt wird aus der Gruppe Silicium-oxid, Aluminiumoxid, Diatomeenerde, Bentonitglas und verschiedene Tone.

7. Alkoxylierungsverfahren nach Anspruch 6, wobei der Katalysatorträger Siliciumoxid und die Katalysatorkomponente Diethylaluminiumfluorid ist.

8. Alkoxylierungsverfahren nach den Ansprüchen 1 bis 7, wobei das Alkoxylierungsmittel gewählt wird aus der Gruppe Ethylenoxid, Propylenoxid und Mischungen hiervon.

9. Alkoxylierungsverfahren nach den Ansprüchen 1 bis 8, wobei das Kontaktieren bei einer Temperatur zwischen etwa 90°C und etwa 200°C durchgeführt wird.

10. Alkoxylierungsverfahren nach den Ansprüchen 1 bis 9, wobei der Alkoxylat-Vorläufer ei Alkohol ist.

**Revendications**

1. Procédé d'alkoxylation comprenant:

la mise en contact dans des conditions d'alkoxylation, à une température comprise entre environ 20°C et environ 260°C, pendant un temps suffisant pour alkoxyler jusqu'au degré désiré,

d'un précurseur d'alkoxylate choisi dans le groupe constitué des polyalcools, des alcools insaturés, des alcools saturés linéaires et remifiés, des alkyl phénols, des aldéhydes, des cétones, des amides, des amines, des acides organiques et des mercaptans; et

d'un agent d'alkoxylation choisi dans le groupe constitué des oxydes d'alkylène alpha et béta, en présence d'un catalyseur acide du type Lewis, caractérisé en ce que ce catalyseur comprend au moins un constituant de catalyseur répondant à la formule générale:

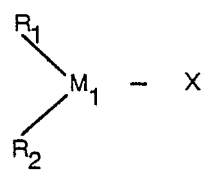 ou 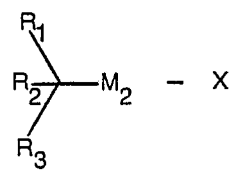

dans laquelle $M_1$ est choisi dans le groupe constitué de l'aluminium, du gallium, de l'indium et du thallium,

$M_2$ est choisi dans le groupe constitué du titane, du zirconium et du hafnium,

X est un halogène choisi dans le groupe constitué du chlore et du fluor, et

$R_1$, $R_2$ et $R_3$ sont choisis indépendamment dans le groupe constitué de l'hydrogène, des groupes alkyles contenant de 1 à 20 atomes de carbone, des groupes alkoxyle contenant de 1 à 20 atomes de carbone et des halogènes, au moins un des symboles $R_1$, $R_2$ ou $R_3$ devant être un groupe alkyle ou alkoxyle.

2. Procédé d'alkoxylation de la revendication 1, dans lequel X est le fluor.

3. Procédé d'alkoxylation suivant les revendications 1 ou 2, dans lequel ce catalyseur est choisi dans le groupe des constituants de catalyseur constitué des fluorures de dialkyl aluminium, des difluorures d'alkyl aluminium, des fluorures de trialkyl zirconium, des difluorures de dialkyl zirconium, des trifluorures

d'alkyle zirconium, des fluorures de trialkyl titane, des fluorures de dialkyl titane, des difluorures d'alkyl titane, des fluorures de dialkoxy aluminium, des difluorures d'alkoxy aluminium, des fluorures de trialkoxy zirconium, des difluorures de dialkoxy zirconium, des trifluorures d'alkoxy zirconium, des fluorures de trialkoxy titane, des difluorures de dialkoxy titane, et des trifluorures d'alkoxy titane, dans lesquels ces groupes alkyle et alkcoxy contiennent de 1 à 14 atomes de carbone.

4. Procédé d'alkoxylation des revendications 1 à 3, dans lequel ce catalyseur est le fluorure de diéthyl aluminium.

5. Procédé d'alkoxylation des revendications 1 à 4, dans lequel ce catalyseur est un catalyseur sur support préparé en mettant en contact ce constituant de catalyseur répondant à cette formule générale avec un support de catalyseur ayant des groupes hydroxyle actifs en surface.

6. Procédé d'alkoxylation de la revendication 5, dans lequel ce support de catalyseur est choisi dans le groupe constitué de la silice, de l'alumine, de la terre de diatomée, de la bentonite, du verre et de diverses argiles.

7. Procédé d'alkoxylation de la revendication 6, dans lequel ce support de catalyseur est la silice et ce constituant de catalyseur est le fluorure de diéthyl aluminium.

8. Procédé d'alkoxylation des revendications 1 à 7, dans lequel l'agent d'alkoxylation est choisi dans le groupe constitué de l'oxyde d'éthylène, de l'oxyde de propylène, et de mélanges de ceux-ci.

9. Procédé d'alkoxylation des revendications 1 à 8, dans lequel cette mise en contact est effectuée à une température comprise entre environ 90°C et environ 200°C.

10. Procédé d'alkoxylation des revendications 1 à 9, dans lequel ce précurseur d'alkoxylate est un alcool.

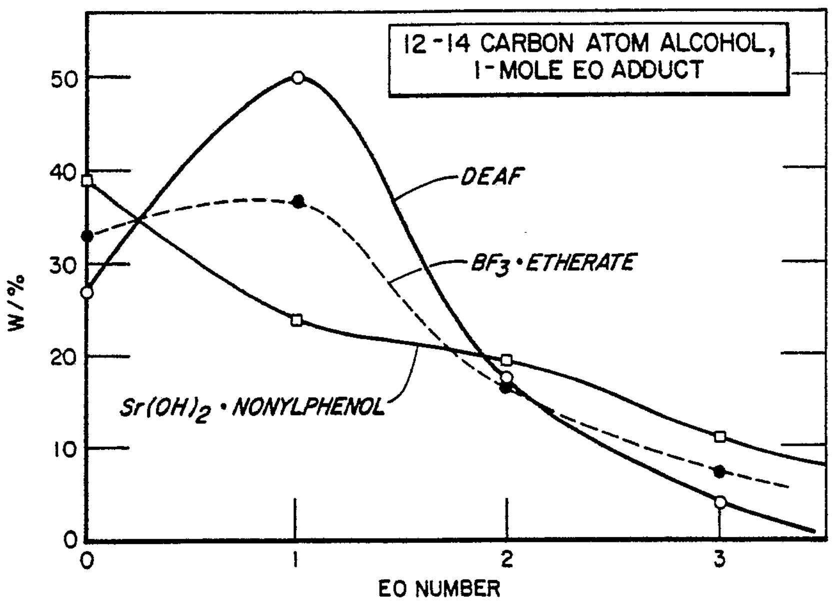
12-14 CARBON ATOM ALCOHOL,
1-MOLE EO ADDUCT
DEAF
$BF_3$ • ETHERATE
$Sr(OH)_2$ • NONYLPHENOL
W/%
0
10
20
30
40
50
0
1
2
3
EO NUMBER

FIG. 1

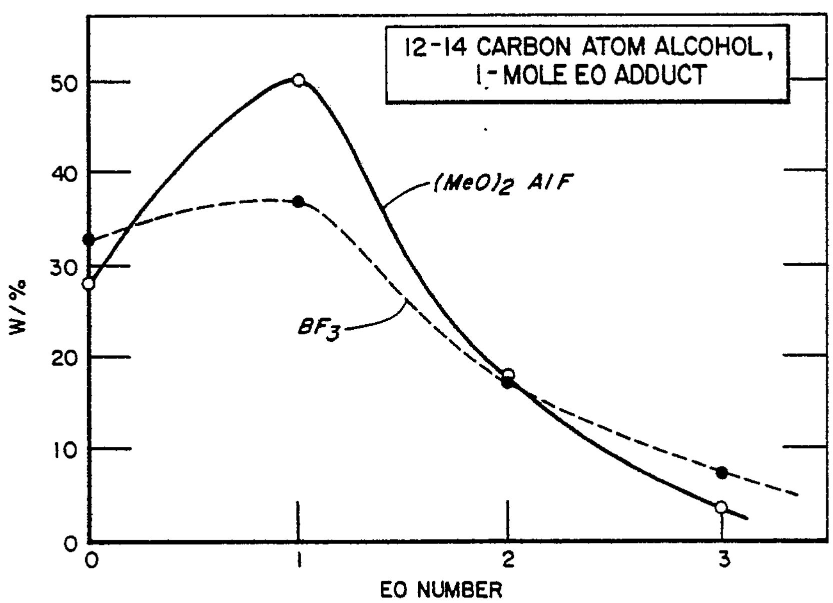
12-14 CARBON ATOM ALCOHOL,
1-MOLE EO ADDUCT
$(MeO)_2$ AlF
$BF_3$
W/%
0
10
20
30
40
50
0
1
2
3
EO NUMBER

FIG. 2

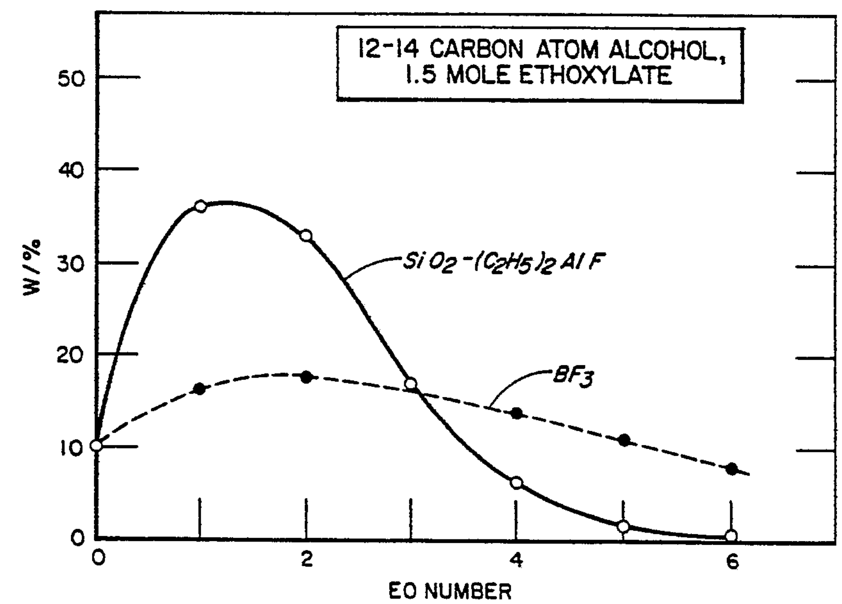
12-14 CARBON ATOM ALCOHOL,
1.5 MOLE ETHOXYLATE
W/%
50
40
30
20
10
0
0
2
4
6
EO NUMBER
$SiO_2-(C_2H_5)_2AlF$
$BF_3$

Fig. 3